# EUROPÄISCHE PATENTSCHRIFT

(11) **EP 0 567 447 B1**
(45) Veröffentlichungstag und Bekanntmachung des Hinweises auf die Patenterteilung: **11.09.1996**
(21) Anmeldenummer: 93890072.7
(22) Anmeldetag: 05.04.1993
(51) Int. Cl.: A61B 5/00

(54) **Sensoranordnung zur direkten oder indirekten Bestimmung physikalischer oder chemischer Parameter**
Sensor arrangement for the direct or indirect determination of the physical or chemical parameter
Arrangement de sonde pour la détermination directe ou indirecte de paramètre physique ou chimique

(30) Priorität: 23.04.1992 AT 838/92
(43) Veröffentlichungstag der Anmeldung: 27.10.1993
(73) Patentinhaber: AVL Medical Instruments AG, CH-8207 Schaffhausen (CH)
(72) Erfinder: Lübbers, Dietrich Werner, Dr., D-4600 Dortmund (DE); Karpf, Hellfried, Dr., A-8043 Graz (AT)
(74) Vertreter: Krause, Walter, Dr. Dipl.-Ing.

(56) Entgegenhaltungen:
- EP-A- 0 121 831
- EP-A- 0 477 501
- US-A- 4 557 900

## Beschreibung

Die Erfindung betrifft eine Sensoranordnung zur direkten oder indirekten, optischen Bestimmung physikalischer oder chemischer Parameter, bestehend aus einem für die zu messende chemische Substanz oder physikalische Größe durchlässigen Indikatorraum, welcher mindestens einen Indikator beinhaltet, sowie aus einer die Anregungsstrahlung für den Indikator liefernde Strahlungsquelle und einem die vom Indikator ausgehende Meßstrahlung erfassenden Detektor, wobei der Kontakt zwischen Strahlungsquelle und Indikator bzw. Indikator und Detektor ausschließlich über die Anregungsstrahlung bzw. die Meßstrahlung erfolgt.

Eine Sensoranordnung der eingangs genannten Art ist beispielsweise aus der DE-A1 30 01 669 bekannt geworden. Um ein Ausschwemmen des Indikators aus dem Indikatorraum zu vermeiden, ist dieser kovalent an eine den Indikatorraum abschließende Membran gebunden. Die Membran kann dadurch für die zu messenden Substanzen besser durchgängig gemacht werden, ohne daß es zu einer vermehrten Freisetzung des Indikators kommt.

Nun ist es bekannt, daß menschliches und tierisches Gewebe, besonders die Haut, für optische Strahlen im roten und infraroten Wellenlängenbereich zwischen ca. 600 nm und 1300 nm relativ gut durchlässig ist. Da es körpereigene Substanzen gibt, die in diesem Bereich charakteristische Absorptionsspektren haben, können diese Substanzen im Inneren des Körpers - gewissermaßen durch ein optisches Fenster - nichtinvasiv erfaßt werden.

Diese Fenster wurden beispielsweise genutzt, um die Sauerstoffsättigung des intravaskulären Hämoglobins fotometrisch zu messen. Mit der heute zur Verfügung stehenden Technik lassen sich z.B. oxygeniertes und deoxygeniertes Hämoglobin innerhalb des Schädels eines Neugeborenen messen, sodaß dadurch eine kontinuierliche Überwachung der Sauerstoffversorgung des Gehirns möglich ist. Auch bei der Pulsoxymetrie, die die arterielle Sauerstoffsättigung des Hämoglobins erfaßt, wird dieses Phänomen des optischen Fensters ausgenützt.

In der EP-A2 0 121 831 wird eine Anordnung zum Messen von diffundierenden Partikeln beschrieben. Um die Messung unter dem Augenlid zu ermöglichen, ist ein Trägerkörper vorgesehen, der Vertiefungen in der am Lid anliegenden Fläche aufweist, in die Optoden eingelegt sind. Solche Optoden bestehen bespielsweise aus Kunststoffolien, in denen Indikatoren angeordnet sind. Die Indikatoren können in der Folie bzw. an deren Oberfläche immobilisiert sein, sodaß beispielsweise toxische Indikatoren bzw. Indikatoren, welche im menschlichen oder tierischen Körper unerwünschte Reaktionen verursachen, nicht verwendet werden können.

In neuerer Zeit werden auch nichtkörpereigene Substanzen in den Körper eingeführt und an der freigelegten Organoberfläche gemessen (VANDERKOOI et al.: The Journal of Biological Chemistry, Vol. 262, No.12 pp.5476-5482, 1987). Im genannten Wellenlängenbereich wird z.B. die Phosphoreszenz eines Sauerstoffindikators, der an Serumalbumin gebunden ist, innerhalb der Blutgefäße gemessen und so die intravaskuläre Sauerstoffkonzentration an der Gehirnoberfläche bestimmt. Verteilungsprobleme im Gewebe sowie Rückwirkungen von Gewebebestandteilen können jedoch Anlaß zu Fehlmessungen sein. Liegen z.B. mehrere Gefäße übereinander, so wird eine exakte Lokalisierung fast unmöglich, da die Lokalisierung durch das Indikatorsignal selbst erfolgt. Ein weiterer Nachteil ist, daß nur Indikatoren angewandt werden können, die nicht toxisch sind und keinerlei biologische Reaktionen auslösen.

Aufgabe der im Anspruch 1 definierten Erfindung ist es, ausgehend von der eingangs beschriebenen Anordnung eine Sensoranordnung vorzuschlagen, mit welcher physikalische oder chemische Parameter in menschlichen oder tierischen Körpern exakter zu erfassen sind, wobei eine genaue Zuordnung des Meßwertes zum Meßort mit hoher regionaler Auflösung möglich und auch toxische Indikatoren zugelassen sein sollen.

Diese Aufgabe wird erfindungsgemäß dadurch gelöst, daß der Indikatorraum von einer eine bestimmte äußere Formgebung aufweisende Kapsel vollständig umschlossen ist, deren Material biokompatibel, sterilisierbar und für die Anregungsstrahlung und die vom Indikator ausgehende Meßstrahlung im Wellenlängenbedie vom Indikator ausgehende Meßstrahlung im Wellenlängenbereich zwischen 600 nm und 1300 nm durchlässig ist, wobei die Kapsel in Magen, Darm, Blutgefäße, Bronchien, Harnblase einbringbar ist und wobei die Kapsel im wesentlichen kugelförmig, linsenförmig oder zylinderförmig mit abgerundeten Enden, faden- oder schlauchförmig ausgeführt ist, bzw. daß die Kapsel zur Implantierung im Gewebe flächig ausgeführt ist. Durch die erfindungsgemäße Maßnahme kann der Ort der Bestimmung des physikalischen oder chemischen Parameters eindeutig definiert werden, da es zu keinen Verdünnungen und unvorhersehbaren Verteilungen des Indikators im Gewebe kommt. Durch die Umschließung des Indikatormaterials durch die Kapsel und der Vermeidung eines direkten Kontaktes zwischen biologischem Gewebe und Indikatormaterial können auch toxische oder gewebeinkompatible Indikatormaterialien zur Anwendung kommen. Falls der Indikator in einer Matrix vorliegt, muß natürlich auch diese im Bereich zwischen 600 nm und 1300 nm durchlässig sein. Der Indikator kann direkt auf den zu messenden Parameter mit einer Änderung seiner optischen Eigenschaften reagieren, oder auch indirekt, d.h. über ein Reaktionsprodukt oder eine Zwischenreaktion. Als Indikatoren eignen sich Lumineszenzindikatoren und Absorptionsindikatoren. Unter biokompatibel wird in diesem Zusammenhang verstanden, daß das Kapselmaterial möglichst biostabil ist und keine wesentlichen Änderungen bzw. Störungen in den biologischen Abläufen des Körpers hervorruft. Die zu messende Substanz kann auch durch selektive Diffusion und Löslichkeit oder durch Carrier im Kapselmaterial angereichert werden, wodurch die Meßgenauigkeit weiter erhöht werden kann.

Die Kapsel kann beispielsweise aus einem im Wellenlängenbereich zwischen 600 nm und 1300 nm durchlässigen Polymer oder auch aus Glas bestehen. In einer weiter unten ausführlicher beschriebenen Ausführungsvariante besteht die Kapsel erfindungsgemäß aus einer Dialysemembran.

Ein zusätzlicher Vorteil besteht darin, wenn die Kapsel ohne Beeinträchtigung ihrer Funktion durch Anwendung von Hitze und ggf. Druck oder Strahlung sterilisierbar ist.

Je nach Anwendung sind verschiedenste äußere Formen der Kapsel denkbar, welche aufgrund des zur Verfügung stehenden Meßfensters im Wellenlängenbereich zwischen 600 nm und 1300 nm im folgenden auch "Fensteroptode" genannt wird.

So ist insbesondere für die Untersuchung des Magen Darmbereiches eine im wesentlichen kugelförmig, linsenformig oder zylinderförmig mit abgerundeten Enden ausgeführte Kapsel denkbar, welche ohne Schwierigkeiten geschluckt werden kann. Beispielsweise wurden bereits Polymerkapseln mit einem Durchmesser kleiner 1 µm hergestellt. Bei räumlichen Messungen kann es vorteilhaft sein, mehrere Indikatoren mit unterschiedlichen optischen Eigenschaften gleichzeitig zu benutzen, die in einer einzigen Kapsel oder in mehreren Kapseln eingelagert sind.

Weiters sind auch faden- oder schlauchförmig ausgebildete Kapseln denkbar bzw. Kapseln, welche flächig ausgeführt sind. Durch Implantation einer oder mehrerer flächig ausgeführter Fensteroptoden kann beispielsweise bei muskulokutanen Transplantaten durch Messung des Gewebe-pH kontrolliert werden, ob eine ordnungsgemäße Sauerstoffversorgung vorliegt. Ein anderer Anwendungsbereich ist z.B. die lokale Messung des pH-Wertes auf der Gehirnoberfläche, beispielsweise nach Gehirnoperationen, bei geschlossener Schädeldecke. Nach Implantation der entsprechenden Kapseln bzw. Fensteroptoden kann die pH-Kontrolle von außen mit geringer Belästigung für den Patienten durchgeführt werden. Da die Implantationswunde geschlossen ist und kein direkter Kontakt über Lichtleiter erforderlich ist, kann das Infektionsrisiko gegenüber dem einer Messung mit einer invasiven Methode minimal gehalten werden.

Die Fensteroptode läßt sich mit einfachen chirurgischen Mitteln fixieren. Beispielsweise können eine oder mehrere kugel-, linsen- oder zylinderformige Kapseln an einen Faden aufgereiht oder mehrere derartige Kapseln an einem flächigen Netzwerk befestigt sein. Nach Beendigung der Messung oder der Meßperiode können die Kapseln wieder entfernt und ihre Meßfunktion überprüft werden.

In einer Weiterbildung der Erfindung ist vorgesehen, daß die Kapsel Teilchen aufweist, welche zur optischen Ortung oder zur Ortung mittels Ultraschall oder Röntgenstrahlen innerhalb des Körpers dienen.

Weiters kann es insbesondere für Messungen im reflektierten Licht von Vorteil sein, wenn die Kapsel zumindest eine reflektierende Fläche aufweist, welche an den Indikator oder eine den Indikator aufnehmende Schicht angrenzt. Der Indikator im Inneren der Kapsel kann beispielsweise in einer Hydrogelschicht vorliegen.

Das Prinzip der Fensteroptode läßt sich für die Messung aller Größen anwenden, für welche geeignete Indikatoren zur Verfügung stehen. Insbesondere ist vorgesehen, daß in der Kapsel zumindest eine Indikatorsubstanz zur Messung der Ionenkonzentration, des Gaspartialdruckes, der Enzym- oder Substratkonzentration, der Ionenstärke, des Druckes oder der Temperatur vorgesehen ist.

Im speziellen seien chemische Größen wie pH, Kalium-, Kalzium-, Natrium- oder Magnesiumionenkonzentration, Partialdrücke von Kohlendioxyd, Sauerstoff oder Ammoniak sowie physikalische Größen wie Druck und Temperatur zu nennen. Große Bedeutung hat auch die Bestimmung der Substrate Glukose, Laktat, Kreatinin u.dgl., welche mit entsprechenden Indikatoren und der beschriebenen Sensoranordung möglich ist.

Die Erfindung wird im folgenden anhand von Zeichnungen näher erläutert. Es zeigen:
- Fig. 1: eine erfindungsgemäße Sensoranordnung in schematischer Darstellung,
- Fig. 2: eine Ausführungsvariante der Sensoranordnung nach Fig. 1,
- Fig. 3 bis 7: Ausführungsvarianten von Kapseln mit eingeschlossenem Indikator, sogenannte "Fensteroptoden" der Sensoranordnungen nach Fig. 1 und 2, und
- Fig. 8: die Anordnung mehrerer Kapseln auf einem Netzwerk.

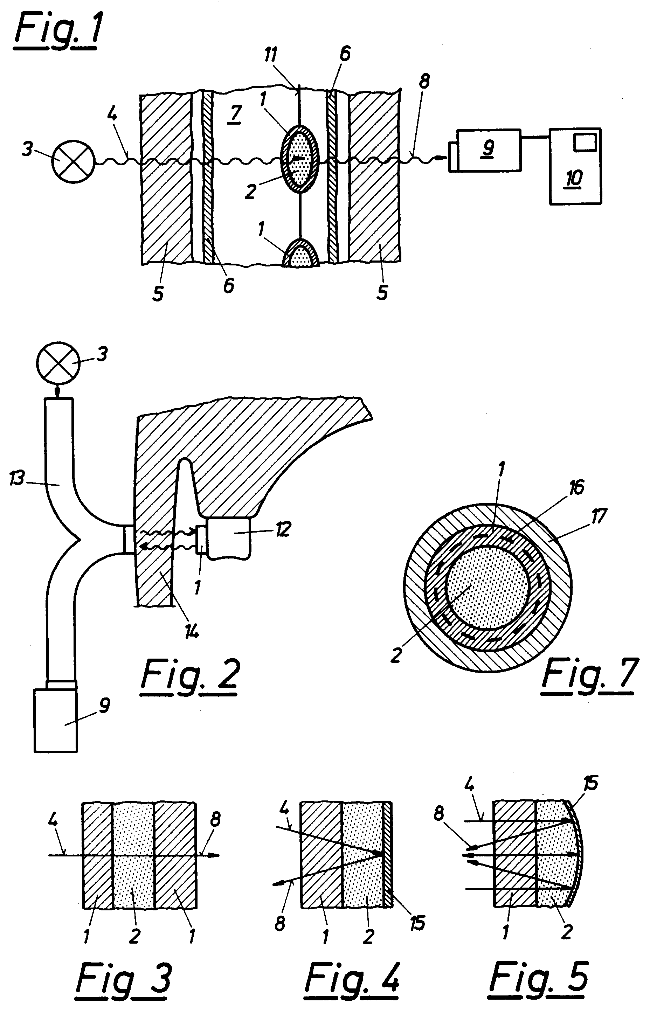

Mit der in Fig. 1 dargestellten Sensoranordnung kann z.B. im Magen eines Kleinkindes eine pH-Messung durchgeführt werden. Dazu wird in den Magen eine Kapsel 1 eingebracht, welche den Indikator 2 umschließt. Die von einer Strahlungsquelle 3 ausgehende Anregungsstrahlung 4, im Wellenlängenbereich zwischen 600 nm und 1300 nm durchdringt das Körpergewebe 5, die Magenwand 6, die im Magenlumen 7 liegende Kapsel 1 und beaufschlagt den Indikator 2. Die vom Indikator 2 ausgehendes Meßstrahlung 8 gelangt nach dem Passieren der Kapsel 1, der Magenwand 6 und des Körpergewebes 5 in einen Detektor 9, welcher mit einer Auswerteeinheit 10 in Verbindung steht. Im dargestellten Beispiel befindet sich im Inneren der Kapsel 1 ein Hydrogel mit dem pH-Indikator. Das Kapselmaterial ist eine für H⁺-Ionen durchlässige Dialysemembran, sodaß ein sich ändernder pH-Wert im Magen auf die optischen Parameter des Indikators einwirkt und eine Änderung in der Meßstrahlung vom Detektor 9 erfaßt werden kann. Die Kapsel 1 kann auch an einem Faden 11 befestigt sein, oder es können mehrere Kapseln 1 auf einen Faden 11 aufgereiht vorliegen, sodaß in definierten Abständen mehrere pH-Messungen durchgeführt werden können. Eine Selektivität der Kapsel 1 bzw. der Kapselmembran für H⁺-Ionen ist nicht unbedingt nötig, wenn man z.B. zwei pH-Indikatoren in der Kapsel vorsieht und die aus Sensors, and Actuaters 4 (1983) 473 bis 479 bekannte Meßmethode benutzt, um die Ionenstärkeempfindlichkeit von Ionenindikatoren zu kontrollieren.

Selbstverständlich können durch die Auswahl entsprechender Indikatoren auch andere Ionen wie Kalium, Kalzium, Natrium oder Magnesium gemessen werden. Es kann ein Absorptions- oder Lumineszenzindikator benutzt werden, dessen optische Eigenschaften der pH-Wert der unmittelbaren Umgebung bestimmt. Mit der Sensoranordnung werden dann je nach verwendetem Indikator entweder Absorptionsänderungen oder Lumineszenzänderungen nach bekannten Verfahren gemessen.

Ein weiteres Beispiel einer nichtinvasiven pH-Messung mit einer IR-Fensteroptode in von außen zugänglichen Körperhöhlen ist in Fig. 2 dargestellt. Die Kapsel 1 zur Messung des pH-Wertes im Mund ist hier an einem Zahn 12 befestigt und die von einem zweiarmigen Lichtleiter 13 transportierte Anregungs-und Meßstrahlung durchdringt die Backe 14 im Bereich der Kapsel 1. Die Anordnung des Lichtleiters 13 wird so gewählt, daß ein Maximum an Signal erfaßt wird. Während bei der Anordnung nach Fig. 1 die Messung mit durchfallendem Licht erfolgt (siehe Detail der Kapsel entsprechend Fig. 3), erfolgt die Messung bei der Anordnung nach Fig. 2 mit reflektiertem Licht, wobei insbesondere bei Verwendung von Absorptionsindikatoren in der Kapsel eine reflektierende Fläche 15 angeordnet ist, welche an den Indikator 2 oder eine den Indikator aufnehmende Schicht angrenzt. In Fig. 4 und Fig. 5 sind Details derartiger Kapseln dargestellt, wobei in Fig. 4 die reflektierende Fläche 15 plan und in Fig. 5 konkav ausgeführt ist. Bei der Anordnung nach Fig. 2 ist die reflektierende Fläche auf der am Zahn 12 anliegenden Seite der Kapsel 1 angeordnet.

Eine ähnliche Meßgeometrie wie in Fig. 2 kann auch zur Messung des O₂- oder CO₂-Partialdruckes in der Nase verwendet werden, wobei eine Fensteroptode mit einem entsprechenden Indikator in der Nase befestigt wird und die Zuleitung der Anregungsstrahlung bzw. Ableitung der Meßstrahlung über eine an der Nase anliegende, brillenartige Vorrichtung erfolgen kann. Durch diese Maßnahme kann sich der Patient völlig frei bewegen und ist auch in der Atmung nicht behindert.

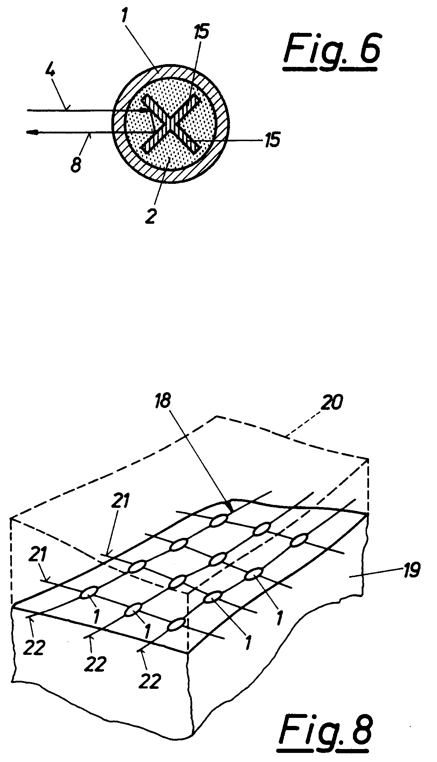

Gemäß Fig. 6 ist es auch möglich, im Inneren der Kapsel 1 mehrere reflektierende Flächen 15 derart anzuordnen, daß die Meßstrahlung 8 parallel zur einfallenden Anregungsstrahlung 4 reflektiert wird.

Um den exakten Meßort im Körper lokalisieren zu können, kann die Kapsel 1 im Inneren aber auch in der Kapselwand Teilchen 16 aufweisen, welche eine optische Ortung oder eine Ortung mittels Ultraschall oder Röntgenstrahlen zulassen. Die in Fig. 7 dargestellte Kapsel 1 weist derartige Teilchen 16 in der Kapselwand auf, wobei die eigentliche Kapsel von einer biokompatiblen Schicht 17 umgeben ist.

Schließlich ist in Fig. 8 die nichtinvasive pH-Messung nach Implantation mehrerer an einem flächigen Netzwerk 18 befestigter Kapseln 1 dargestellt. Damit kann beispielweise durch Messung des Gewebe-pH kontrolliert werden, ob eine ordnungsgemäße Sauerstoffversorgung zwischen dem Gewebe 19 und dem Transplantat 20 vorliegt. Die Form der implantierten Fenstroptoden läßt sich, wie gesagt, der Struktur des Gewebes in dem die Implantation erfolgt, anpassen, so könnte beispielsweise anstelle des Netzwerkes 18 für kleinere Bereiche auch flächige Optode verwendet werden. Wenn man beispielsweise für die Querfäden 21 des Netzwerkes 18 vom Körper resorbierbares Material verwendet, können die Kapseln bei geeigneter Form mit Hilfe der Längsfäden 22 am Ende der Meßserie aus dem Gewebe gezogen werden.

## Patentansprüche

1. Sensoranordnung zur direkten oder indirekten, optischen Bestimmung physikalischer oder chemischer Parameter, bestehend aus einem für die zu messende chemische Substanz oder physikalische Größe durchlässigen Indikatorraum, welcher mindestens einen Indikator (2) beinhaltet, sowie aus einer die Anregungsstrahlung (4) für den Indikator (2) liefernde Strahlungsquelle (3) und einem die vom Indikator (2) ausgehende Meßstrahlung (8) erfassenden Detektor (9), wobei der Kontakt zwischen Strahlungsquelle und Indikator (2) bzw. Indikator (2) und Detektor (9) ausschließlich über die Anregungsstrahlung (4) bzw. die Meßstrahlung (8) erfolgt, wobei der Indikatorraum von einer eine bestimmte äußere Formgebung aufweisende Kapsel (1) vollständig umschlossen ist, deren Material biokompatibel, sterilisierbar und für die Anregungsstrahlung (4) und die vom Indikator (2) ausgehende Meßstrahlung (8) im Wellenlängenbereich zwischen 600 nm und 1300 nm durchlässig ist, wobei die Kapsel (1) in Magen, Darm, Blutgefäße, Bronchien, Harnblase einbringbar ist und wobei die Kapsel (1) im wesentlichen kugelförmig, linsenförmig oder zylinderförmig mit abgerundeten Enden, faden- oder schlauchförmig ausgeführt ist, bzw. daß die Kapsel (1) zur Implantierung im Gewebe flächig ausgeführt ist.

2. Sensoranordnung nach Anspruch 1, **dadurch gekennzeichnet**, daß die Kapsel (1) Teilchen (16) aufweist, welche zur optischen Ortung oder zur Ortung mittels Ultraschall oder Röntgenstrahlen innerhalb des Körpers dienen.

3. Sensoranordnung nach Anspruch 1, **dadurch gekennzeichnet**, daß die Kapsel (1) zumindest eine reflektierende Fläche (15) aufweist, welche an den Indikator oder eine den Indikator (2) aufnehmende Schicht angrenzt.

4. Sensoranordnung nach einem der Ansprüche 1 bis 3, **dadurch gekennzeichnet**, daß der Indikator (2) im Inneren der Kapsel (1) in einer Hydrogelschicht vorliegt.

5. Sensoranordnung nach Anspruch 1, **dadurch gekennzeichnet**, daß eine oder mehrere kugel-, linsen- oder zylinderförmige Kapseln (1) an einem Faden (11) aufgereiht sind.

6. Sensoranordnung nach Anspruch 1, **dadurch gekennzeichnet**, daß mehrere kugel-, linsen- oder zylinderförmige Kapseln (1) an einem flächigen Netzwerk befestigt sind.

7. Sensoranordnung nach einem der Ansprüche 1 bis 6, **dadurch gekennzeichnet**, daß die Kapsel (1) aus Polymer oder Glas besteht.

8. Sensoranordnung nach Anspruch 7, **dadurch gekennzeichnet**, daß die Kapsel (1) aus einer Dialysemembran besteht.

9. Sensoranordnung nach einem der Ansprüche 1 bis 8**, dadurch gekennzeichnet**, daß in der Kapsel (1) zumindest eine Indikatorsubstanz zur Messung der Ionenkonzentration, des Gaspartialdruckes, der Enzym- oder Substratkonzentration, der Ionenstärke, des Druckes oder der Temperatur vorgesehen ist.

## Claims

1. A sensor arrangement for direct or indirect optical determination of physical or chemical parameters, comprising an indicator cell, which is permeable to the chemical substance or physical quantity to be measured and contains at least one indicator (2), and further comprising a radiation source (3) supplying the excitation radiation (4) for the indicator (2), and a detector (9) for detection of the response radiation (8) emitted by the indicator (2), where the contact between the radiation source (3) and the indicator (2), or the indicator (2) and the detector (9) is exclusively established via the excitation radiation (4) and the response radiation (8), and where the indicator cell is completely enclosed by a capsule (1) of a defined shape, whose material is biocompatible, sterilizable and transparent to the excitation radiation (4) and to the response radiation (8) emitted by the indicator (2) in the range of wavelengths between 600 and 1,300 nm, and where the capsule (1) may be introduced into stomach, intestine, blood vessels, bronchial tubes, urinary bladder, and where the capsule (1) is configured as an essentially spherical, lenticular, or cylindrical structure with rounded edges, or as a string-like or tubular structure, or as a sheet structure for implantation in tissue.

2. A sensor arrangement as in claim 1, **characterized in that** the capsule (1) is provided with particles (16) permitting its localization inside the body, which may be effected by optical means or ultrasonic techniques or X-rays.

3. A sensor arrangement as in claim 1, **characterized in that** the capsule (1) is provided with at least one reflecting surface (15), which is adjacent to the indicator (2) or a layer containing the indicator (2).

4. A sensor arrangement as in any of claims 1-3, **characterized in that** the indicator (2) inside the capsule (1) is embedded in a hydrogel layer.

5. A sensor arrangement as in claim 1, **characterized in that** one or several capsules (1) of spherical, lenticular or cylindrical shape are attached on a string (11).

6. A sensor arrangement as in claim 1, **characterized in that** several capsules (1) of spherical, lenticular or cylindrical shape are attached to a two-dimensional network (18).

7. A sensor arrangement as in any of claims 1-6, **characterized in that** the capsule (1) is made of polymer material or glass.

8. A sensor arrangement as in claim 7, **characterized in that** the capsule (1) is made of a dialytic membrane.

9. A sensor arrangement as in any of claims 1-8, **characterized in that** at least one indicator substance is provided in the capsule (1) for measuring ionic concentration, gas partial pressure, enzyme or substrate concentration, ionic strength, pressure, or temperature.

## Revendications

1. Capteur pour déterminer directement ou indirectement de manière optique des paramètres physiques ou chimiques, comprenant une cavité d'indicateur perméable à la substance chimique à mesurer ou à la grandeur physique, et qui contient au moins un indicateur (2), ainsi qu'une source de rayonnement (3) fournissant le rayonnement d'excitation (4) pour l'indicateur (2) et un détecteur (9) détectant le rayonnement de mesure (8) émis par l'indicateur (2), le contact entre la source de rayonnement et l'indicateur (2) ou l'indicateur (2) et le détecteur (9) se faisant exclusivement par le rayonnement d'excitation (4) et le rayonnement de mesure (8), caractérisé en ce que la chambre d'indicateur est entourée complètement par une capsule (1) ayant une certaine forme extérieure, dont la matière est biocompatible, peut être stérilisée et est transparente au rayonnement d'excitation (4) et au rayonnement de mesure (8) émis par l'indicateur (2), dans une plage de longueur d'onde comprise 600 nm et 1300 nm, la capsule (1) pouvant s'introduire dans l'estomac, l'intestin, des vaisseaux sanguins, les bronches, la vessie et la capsule (1) étant réalisée essentiellement sous forme sphérique, lenticulaire ou cylindrique avec des extrémités arrondies, des fils ou de forme tubulaire, et la capsule (1) est réalisée plate pour implantation dans le tissu.

2. Capteur selon la revendication 1, caractérisé en ce que la capsule (1) comporte des particules (16) servant à la localisation optique ou à la localisation par ultrasons ou par rayonnement X à l'intérieur du corps.

3. Capteur selon la revendication 1, caractérisé en ce que la capsule (1) présente au moins une surface réfléchissante (15) adjacente à l'indicateur ou à une couche recevant l'indicateur (2).

4. Capteur selon l'une des revendications 1 à 3, caractérisé en ce que l'indicateur (2) est présent à l'intérieur de la capsule (1) dans une couche d'hydrogel.

5. Capteur selon la revendication 1, caractérisé en ce qu'une ou plusieurs capsules sphériques, lenticulaires ou cylindriques (1) sont enfilées sur un fil (11).

6. Capteur selon la revendication 1, caractérisé en ce que plusieurs capsules sphériques, lenticulaires ou cylindriques (1) sont fixées sur un réseau formant une surface.

7. Capteur selon l'une des revendications 1 à 6, caractérisé en ce que la capsule (1) est en polymère ou verre.

8. Capteur selon la revendication 7, caractérisé en ce que la capsule (1) est une membrane de dialyse.

9. Capteur selon l'une des revendications 1 à 8, caractérisé en ce que la capsule (1) contient au moins une substance indicatrice pour mesurer la concentration en ions, la pression partielle d'un gaz, la concentration en enzyme et en substrat, l'intensité en ions, la pression ou la température.
